# EUROPÄISCHE PATENTANMELDUNG

(11) **EP 2 409 766 A1**
(43) Veröffentlichungstag der Anmeldung: **25.01.2012**
(21) Anmeldenummer: 10170613.3
(22) Anmeldetag: 23.07.2010
(51) Int. Cl.: B01L 3/00, G01N 33/543

(54) **Verfahren zur Hydrophilisierung von Oberflächen fluidischer Komponenten und derartige Komponenten enthaltende Bauteile**

(71) Anmelder: F. Hoffmann-La Roche AG, 4070 Basel (CH); Roche Diagnostics GmbH, 68305 Mannheim (DE)
(72) Erfinder: HOFMANN, Wolfgang, 8055, Graz (AT); NOORMOFIDI, Taghi, 8054, Graz (AT); ZAHRL, Doris, 8020, Graz (AT)
(74) Vertreter: Babeluk, Michael

(57) **Zusammenfassung**

Die Erfindung betrifft ein Verfahren zur zumindest teilweisen Belegung der inneren Oberflächen von fluidischen Komponenten, insbesondere eines zumindest ein Sensorelement aufweisenden Messkanals, einer in einen Analysator austauschbar einsetzbaren Sensorkassette mit einem hydrophilen Polymer. Das Verfahren ist dadurch gekennzeichnet dass eine ungebrauchte Sensorkassette in den Analysator eingesetzt wird, dass eine wässrige Lösung eines hydrophilen Polyaminosaccharids (vorzugsweise Chitosan) in den Messkanal der Sensorkassette eingeleitet wird, sowie dass die wässrige Lösung des Polyaminosaccharids nach einer Verweilzeit durch ein gasförmiges oder flüssiges Medium ersetzt wird, wobei Reste des Polyaminosaccharids auf der inneren Oberfläche des Messkanals verbleiben und diese hydrophilisieren.

## Beschreibung

Die Erfindung betrifft ein Verfahren zur zumindest teilweisen Belegung der inneren Oberflächen von fluidischen Komponenten, insbesondere eines zumindest ein Sensorelement aufweisenden Messkanals, einer in einen Analysator einsetzbaren Sensorkassette mit einem hydrophilen Polymer. Weiters betrifft die Erfindung eine Sensorkassette, die in einen Analysator austauschbar einsetzbar ist, deren innere Oberflächen der fluidischen Komponenten, insbesondere des mindestens ein Sensorelement enthaltendenden Messkanals, zumindest teilweise mit einem hydrophilen Polymer belegt ist.

Insbesondere bei der Bestimmung von gasförmigen Analyten (O₂, CO₂) in wässrigen Flüssigkeiten können Probleme bei der Probenmessung bzw. bei der Kalibrierung oder Qualitätskontrolle auftreten, wenn die Probe bzw. das Qualitätskontroll- oder Kalibriermittel den flüssigkeitsführenden Bereich des sensorischen Elements nur unvollständig füllt oder wenn sich dort Gasblasen, beispielsweise Luftblasen, befinden. Gasblasen bilden sich insbesondere bei Vorliegen von uneinheitlichen inneren Oberflächen innerhalb des Messkanals der Sensorkassette, welche unterschiedliche Benetzungseigenschaften durch Flüssigkeiten aufweisen. Besonders häufig kommt es an den Stellen des Messkanals zur Bildung oder Festsetzung von Gasblasen, an welchen ein sprunghafter Übergang in den Benetzungseigenschaften der inneren Oberflächen der verschiedenen fluidischen Komponenten bzw. Bauteile vorhanden ist. Dies ist beispielsweise dann der Fall, wenn Oberflächen aus unterschiedlichen Materialien aufeinander stoßen. Der Messkanal besteht im Allgemeinen aus vielen einzelnen fluidischen Bauteilen aus unterschiedlichen Materialien, deren aneinander grenzende Oberflächen sich durch eine unterschiedliche Hydrophilie oder Hydrophobie auszeichnen und infolge dessen unterschiedliche Benetzungseigenschaften besitzen.

In der US 4,358,423 A (Nedetzky) wird bereits auf das Problem von eingeschlossenen Luftblasen hingewiesen, welche das Messresultat verfälschen, da die Luftblasen eine ausreichende Benetzung der Oberfläche der jeweils eingesetzten Sensorelemente verhindern. Maßnahmen, die einen derartigen Fehler erkennen, sind insbesondere bei automatisch arbeitenden Analysatoren notwendig, bei welchen der Füllvorgang der Messkapillare bzw. die Blasenfreiheit der Probe in der Messkammer automatisch kontrolliert werden muss. Zur Lösung des Problems wird hier ein Verfahren vorgeschlagen, bei welchem der Wert des elektrischen Widerstandes zwischen wenigstens zwei Stellen in der Messkammer gemessen wird, wobei der Füllvorgang der Messkammer in Abhängigkeit der festgestellten Größe des gemessenen Widerstandes gesteuert wird.

Die EP 0 379 156 beschreibt Beschichtungsverfahren, bei welchen zunächst auf die Oberfläche einer medizinischen Vorrichtung, insbesondere eines Katheders eine Polyisocyanatlösung aufgebracht wird, diese (optional) eingetrocknet wird und anschließend eine weitere Lösung eines carboxylsäurehaltigen Polymers aufgebracht wird. Bei solchen zwei- oder mehrstufigen Verfahren mit mehreren beteiligten Reagenzienlösungen und chemischen Reaktionsschritten sind meist viele Prozessschritte notwendig, die anwenderseitig nicht oder nur mit großem Aufwand durchführbar sind.

Eine Beschichtung von Oberflächen medizinischer Implantate, Katheter und Herzschrittmacher mit chitosanhältigen Schichten ist beispielsweise aus der US 5,578,073 A bekannt, erfolgt hier jedoch zur Verringerung des Thromboserisikos. Die Schicht besteht aus Chitosan und einer zusätzlichen, biologisch aktiven Komponente, beispielsweise PVA oder aus Serumalbumin, welches in eine Chitosanmembran eingebettet ist. Derartige Schichten sind allerdings für Messkanäle mit Sensorelementen ungeeignet.

Die US 4,752,426 beschreibt Verfahren zur Hydrophilisierung von Oberflächen, bei welchen zunächst mittels einer Niedertemperatur-Plasmabehandlung chemisch reaktive Gruppen bzw. Radikale auf der Oberfläche erzeugt werden. Anschließend wird eine Monomer-Lösung auf diese Oberfläche aufgebracht. Die Monomere reagieren dort chemisch mit den chemisch reaktiven Gruppen bzw. Radikalen auf der Oberfläche, sodass letztlich auf der Oberfläche mittels Pfropfpolymerisation eine Beschichtung gebildet wird. Nachteilig bei diesen Verfahren ist, dass diese Verfahrensschritte und -bedingungen möglichst exakt aufeinander abgestimmt werden müssen. Beispielsweise müssen die Plasmabehandlungsparameter derartig gewählt werden, dass möglichst nur solche chemisch reaktive Gruppen bzw. Radikale auf der Oberfläche gebildet werden, welche auch als Polymerisationskeime für die nachfolgende Pfropfpolymerisation dienen können.

In der EP 1 595 605 B1 erfolgt die Lösung des Benetzungsproblems durch Bereitstellung eines fluidischen Systems (z.B. eine Sensorkassette) für einen Analysator, welches ein oder mehrere fluidische Bauteile (z.B. Messkanal) und zumindest ein Sensorelement enthält, auf deren inneren Oberflächen direkt ohne weitere Zwischenschichten ein Film eines hydrophilen Polymers aufgebracht wird. Dabei wird zunächst die innere Oberfläche des fluidischen Systems einer physikalischchemischen Vorbehandlung unterzogen. Anschließend werden die inneren Oberflächen der Bauteile mit einer Lösung desselben hydrophilen Polymers in Kontakt gebracht und danach die Lösung durch ein gasförmiges Medium ersetzt, wobei die Oberflächen mit einem Teil der Lösung benetzt bleiben. Schließlich entsteht auf den inneren Oberflächen ein Film des hydrophilen Polymers durch Entzug des Lösungsmittels. Das relativ aufwändige Beschichtungsverfahren kann nur werkseitig durchgeführt werden.

In vielen Anwendungsfällen wäre es allerdings von Vorteil keine werkseitige Beschichtung bzw. Hydrophilisierung der inneren Oberflächen des Messkanals durchzuführen, da zur Vermeidung von Alterungseinflüssen die Hydrophilisierung erst kurz vor der tatsächlichen Verwendung der Kassette in einem Analysator erfolgen soll. Bei der Verwendung von wässrigen Lösungen kann es zudem werkseitig zu unerwünscht frühzeitigen Reaktionen einzelner Sensorelemente mit der wässrigen Lösung kommen, z.B. einer Aktivierung des Sensorelementes durch Aufnahme von Wasser ("wet up" des Sensors).

Für eine ausreichende Hydrophilisierung wäre auch der Einsatz eines hochwirksamen Detergens in einer der Betriebsflüssigkeiten denkbar. Obwohl die prinzipielle Wirksamkeit dieser Maßnahme bewiesen ist, ist deren Einsatz im Rahmen der vorliegenden Erfindung aber aufgrund von Nebeneffekten nicht angebracht.

Die Aufgabe der Erfindung besteht in der Bereitstellung einer Sensorkassette mit hydrophilisierten Oberflächen des Messkanals zwecks Vermeidung von Gasblasenbildung bzw. Anhaftung von Gasblasen bei Befüllung mit wässrigen Betriebsflüssigkeiten, wobei die Hydrophilisierung anwenderseitig kurz vor oder während des Gebrauchs der im Analysator vorliegenden Sensorkassette erfolgen soll.

Diese Aufgabe wird erfindungsgemäß dadurch gelöst, dass
a. eine (neue, ungebrauchte) Sensorkassette in den Analysator eingesetzt wird,
b. eine wässrige Lösung eines hydrophilen Polyaminosaccharids in den Messkanal der Sensorkassette eingeleitet wird, sowie
c. die wässrige Lösung des Polyaminosaccharids nach einer Verweilzeit durch ein gasförmiges oder flüssiges Medium ersetzt wird, wobei Reste des Polyaminosaccharids auf der inneren Oberfläche des Messkanals und den Messkanal begrenzenden Teilen der Sensorelemente verbleiben und diese hydrophilisieren.

Das Verfahren kann auf einfache Weise anwenderseitig - ohne jede Vorbehandlung - bevorzugt als automatische Routine des Analysators durchgeführt werden.

Weiters ist es erfindungsgemäß möglich, bereits in Verwendung stehende Sensorkassetten in vorgegebenen Zeitabständen automatisch oder manuell ausgelöst den Schritten b. und c. zu unterwerfen, um die Hydrophilisierung der Kassette wieder aufzufrischen.

Als Polyaminosaccharid-Lösung wird bevorzugt Chitosan in wässriger Lösung eingesetzt.

Gegenstand der Erfindung ist auch die Verwendung von Chitosan zur Herstellung eines inselartigen oder vollflächigen Belags auf inneren Oberflächen der fluidischen Komponenten von Sensorkassetten, insbesondere eines Messkanals, der zumindest ein Sensorelement aufweist. Überraschenderweise hat sich gezeigt, dass ein Chitosanbelag auf den Sensorelementen die Sensorfunktion nicht beeinträchtigt, insbesondere dann wenn ein inselartiger Belag oder ein Monolayer vorliegen.

Die Erfindung wird im Folgenden anhand von Ausführungsbeispielen näher erläutert. Es zeigen:
- Fig. 1: einen Analysator mit einem eingesetzten Fluidpack (Reagenzienkassette) mit mehreren Behältnissen zur Aufnahme der Betriebsflüssigkeiten und einer Chitosanlösung in einer schematischen Darstellung;
- Fig. 2: die Strukturformel von Chitosan; sowie
- Fig. 3: ein Zustandsdiagramm einer Chitosanlösung, wobei auf der Abszisse der pH-Wert und auf der Ordinate der Chitosangehalt der Lösung aufgetragen ist,

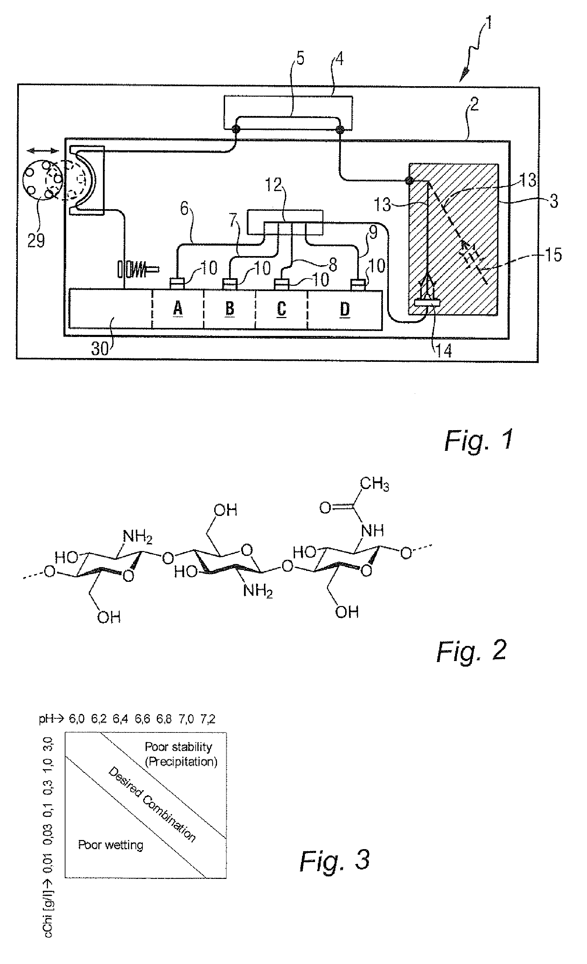

Der in Fig. 1 schematisch dargestellte Analysator 1 zur Analyse von medizinischen Probenflüssigkeiten, beispielsweise von Blutproben, weist eine Reagenzienkassette bzw. ein Fluidpack 2 auf, das austauschbar in den Analysator 1 einsetzbar ist. Im Fluidpack 2 sind mehrere als Reagenzienbeutel ausgeführte Behältnisse A bis D angeordnet, welche Betriebsflüssigkeiten, wie beispielsweise Kalibrier-, Qualitätskontroll-, und Waschflüssigkeiten beinhalten, die wahlweise einer Eingabeeinrichtung 3 und in weiterer Folge einem in einer Sensorkassette 4 angeordneten Messkanal 5 mit zumindest einem Sensorelement zugeführt werden können. Die Eingabeeinrichtung 3 des Analysators 1 weist ein verschwenkbares Eingabeelement 13 (beispielsweise eine Hohlnadel) auf, das in einer Grundposition mit einem Andockelement 14 zur Zufuhr von Kalibier- und Waschmittel in Verbindung steht, wobei in einer aus der Grundposition ausgeschwenkten Stellung 15 Probenflüssigkeiten zugeführt werden können. Die Probeneingabe kann aus unterschiedlichen Gefäßen (z.B. Spritze, Kapillare, Glasgefäß, etc.) erfolgen.

Im vorliegenden Beispiel enthält eine der Wasch-, Kalibrier- und/oder Qualitätskontrollflüssigkeiten in den Behältnissen A bis C zusätzlich Chitosan, oder das Fluidpack 2 weist ein weiteres Behältnis D zur Aufnahme einer vorzugsweise physiologischen Chitosanlösung auf. Diese Ausführungsform ist besonders bevorzugt, da hierdurch das erfindungsgemäße Verfahren automatisiert durchgeführt werden kann. Alternativ ist es auch möglich, die Chitosanlösung auch anderweitig in den Messkanal der Sensorkassette einzuleiten, beispielsweise durch Ansaugen einer chitosanhaltigen Lösung durch das Eingabeelement 13 des Analysators.

Jeder Reagenzienbeutel A bis D weist direkt bei der Einmündung der jeweiligen Anschlussleitung 6, 7, 8, 9 ein vom Analysator ansteuerbares Mehrwegeventil 10 (Beutelventil) mit zumindest zwei Ventilstellungen auf, wobei die erste Ventilstellung eine Fluidverbindung zwischen der jeweiligen Anschlussleitung 6, 7, 8, 9 und dem zugehörigen Reagenzienbeutel A bis D herstellt. In der zweiten Ventilstellung wird der jeweilige Reagenzienbeutel A bis D verschlossen und ein Zugang zur Umgebungsluft hergestellt. Alle von den Mehrwegeventilen 10 wegführenden Anschlussleitungen 6, 7, 8, 9 der Reagenzienbeutel A bis D münden in eine gemeinsame Sammelleitung 12, welche eine Verbindung zum Andockelement 14 der Probeneingabeeinrichtung 3 herstellt.

Die Fluidleitung führt nach der Sensorkassette 4 über den feststehenden Teil einer im Analysator 1 integrierten Schlauchpumpe 29 und mündet in einen in der Reagenzienkassette 2 angeordneten Abfallbeutel 30.

Zusammenfassend stehen somit folgende Lösungen zur Verfügung:
• die Bereitstellung einer chitosanhaltigen Lösung mit physiologischem pH-Wert, vorzugsweise im (ohnehin vorhandenen) Fluidpack des Analysators
• die Bereitstellung eines Verfahrens zur zumindest teilweisen Belegung der inneren Oberflächen des Messkanals mit Chitosan kurz vor oder während des Gebrauchs der Sensorkassette.
• die Bereitstellung einer Sensorkassette wobei der Messkanal oder andere fluidische Komponenten der Sensorkassette zwecks Hydrophilisierung an deren inneren Oberflächen einen inselartigen oder vollflächigen Belag aufweisen, der Chitosan enthält oder aus Chitosan besteht.

Zur Belegung der inneren Oberflächen des Messkanals mit Chitosan wird zunächst der Messkanal mit einer wässrigen Lösung von Chitosan befüllt und anschließend die Lösung durch ein gasförmiges Medium (Luft) ersetzt, wobei Chitosanreste auf den inneren Oberflächen des Messkanals und der Sensorelemente verbleiben.

Alternativ wird zunächst der Messkanal mit einer wässrigen Lösung von Chitosan befüllt und anschließend durch eine Lösung ersetzt, die einen im Vergleich zur Chitosanlösung höheren pH-Wert aufweist, z.B. eine schwach alkalische Betriebsflüssigkeit (Kalibrier-, Qualitätskontroll- oder Waschflüssigkeit) des Analysators. Durch die pH-Erhöhung werden die protonierten Aminogruppen von Chitosan deprotoniert, wodurch sich die Zahl der positiven Ladungen verringert. Die dadurch bewirkte verringerte Löslichkeit von Chitosan begünstigt die Ablagerung an den inneren Oberflächen des Messkanals.

Alternativ kann die chitosanhaltige Lösung auch als Betriebsflüssigkeit (Kalibrierflüssigkeit, QC-Flüssigkeit, Waschflüssigkeit etc.) ausgeführt sein, indem den bestehenden Inhaltsstoffen der Betriebsflüssigkeit noch Chitosan (und evtl. optional noch weitere Substanzen, wie Puffersubstanzen) zugesetzt wird.

Die wässrige Chitosanlösung weist einen pH-Wert < 7, vorzugsweise einen pH-Wert zwischen 6,4 und 6,8, auf,

Die derart behandelten Oberflächen des Messkanals weisen für wässrige Lösungen eine Oberflächenbenetzbarkeit auf, die für alle den Messkanal bildenden Oberflächen gleichartig ist, und meist höher ist als die Oberflächenbenetzbarkeit der inneren Oberflächen des Messkanals ohne das Vorhandensein des Chitosanbelags. Durch die verbesserte und vereinheitlichte Benetzbarkeit für wässrige Flüssigkeiten ist das Risiko von Gasblasenbildung während des Befüllvorgangs mit Betriebs- und Messflüssigkeiten erheblich vermindert.

Wie aus Fig. 2 ersichtlich ist Chitosan ein Polyaminosaccharid welches sich vom Chitin ableitet. Liegen im Gesamtmolekül überwiegend deacetylierte 2-Amino-2-desoxy-β-D-glukopyranose-Einheiten vor, spricht man von Chitosan. Es ergibt sich ein lineares Polymer, das aus etwa 2000 Monomeren besteht.

Der pKs-Wert der Aminogruppen liegt bei 6,5. Das bedeutet, dass bei niedrigeren pH-Wert Chitosan positiv geladen und gut wasserlöslich ist (Polykation). Bei höheren pH-Werten nimmt der Anteil der protonierten Aminogruppen ab. Daraus erklärt sich, dass Chitosan in Säuren bevorzugt löslich ist.

Die pH Abhängigkeit der Chitosan-Löslichkeit wirkt sich positiv auf die Abscheidung des Chitosans aus, da die schrittweise Substitution der Chitosanlösung durch eine der Betriebslösungen eine leichte Alkalisierung bewirkt, die sich positiv auf die Abscheidung des Polysaccharids auswirkt.

### Herstellung der Chitosanlösung:

Die Herstellung der Chitosanlösung erfolgt werkseitig nach folgendem Schema:
• Auflösen von Chitosan in Säure
• Stellen auf einen bestimmten pH-Wert mit Hilfe von Puffersubstanzen

Als saure und basische Komponenten kommen Mineralsäuren und alle Basen in Frage, wobei zur Etablierung eines Puffersystems organische HEPES-Base (als Hepes-Na Salz) vorteilhaft ist.

Als Chitosan-Rohstoff wird das Produkt Sigma #448877 eingesetzt, spezifiziert mit einem Deacetylierungsgrad zwischen 75 und 100 %. Das Produkt wird weiters durch rheologische Parameter spezifiziert.

Als wirksam hat sich beispielsweise folgende Rezeptur erwiesen (die angegebenen Konzentrationen sind die Konzentrationen der finalen Lösung im Endvolumen):

| | |
|---|---|
| Chitosan | ca.0,3 g/l |
| NaCl | 49 mmol/l |
| Hepes, freie Säure | 15 mmol/l |
| Hepes, Natriumsalz | 3 mmol/l |

Weiters kann die Lösung ein Konservierungsmittel enthalten.

Der pH-Wert dieser Lösung liegt z.B. bei 6,7. Bei der Rezepturerstellung hat sich herausgestellt, dass die Eignung der Lösung nicht nur von der Chitosankonzentration, sondern auch stark vom eingestellten pH-Wert abhängt. Bei hohem pH-Wert wird die Lösung instabil aufgrund von Ausfällung des Polymers. Bei zu niedrigem pH-Wert der Lösung kommt es nur zu einer unzureichenden Belegung der inneren Oberflächen. Die Abhängigkeiten entsprechen sinngemäß dem in Fig. 3 dargestellten Schema, wobei auf der Abszisse der pH-Wert und auf der Ordinate der Chitosangehalt aufgetragen ist.

Die Chitosan Lösung befindet sich bevorzugt im Reagenzienpack 2 des Analysators, und zwar in einem beutelartigen Behältnis D gemäß Fig. 1 (siehe auch EP 2 077 452 A1)

Die im Behältnis D des Fluidpacks 2 vorliegende Chitosanlösung wird durch einen automatisierten Ablauf in den Messkanal 5 der Sensorkassette 4 gesaugt und verbleibt dort für eine kurze Verweilzeit von beispielsweise 5 bis 30 s, wobei die inneren Oberflächen des Messkanals 5 mit Chitosan zumindest teilweise belegt werden.

Entweder wird nun der Messkanal 5 leer gesaugt und die Sensorkassette ist dann bereit für die bestimmungsgemäße Verwendung, oder die Chitosanlösung wird direkt durch eine der Betriebsflüssigkeiten mit einem etwas höheren pH-Wert ersetzt, wobei es aufgrund der laminaren Strömung zu einer schrittweisen Vermischung der beiden Lösungen kommt. Diese Vermischung wirkt sich aufgrund des steigenden pH-Wertes positiv auf die Abscheidung des Chitosan an den inneren Oberflächen des Messkanals aus.

Das Verfahren zur zumindest teilweisen Belegung der inneren Oberflächen des Messkanals der Sensorkassette mit Chitosan wird vor dem erstmaligen Gebrauch bzw. Aktivierung einer Sensorkassette durchgeführt.

Weiters ist auch vorgesehen das Verfahren während der 'in-use' Lebensdauer der Sensorkassette wiederholt durchzuführen um die zumindest teilweise Belegung der inneren Oberflächen des Messkanals der Sensorkassette mit Chitosan aufrecht zu erhalten bzw. zu erneuern.

Es hat sich gezeigt, dass die Benetzungswirkung von Chitosan zumindest für einen gewissen Zeitraum persistent ist. Die Deposition des Polysaccharides bewirkt eine zumindest zeitlich begrenzte Hydrophilisierung der Oberflächen des Sensorkanals.

Die in der folgenden Tabelle zusammengefassten Daten zeigen die Wirkung von Benetzungslösungen in einem beispielhaften Messsystem. Dabei ist ein Probenkanal mit einem Sensorarray (siehe beispielsweise WO 2009/062940) eingesetzt. Zwischen den Sensoren befinden sich Metallkontakte, die im Normalfall dazu dienen, aufgrund elektrischer Kontaktierung Proben zu erkennen. Das System kann auch dazu herangezogen werden um Luftblasen innerhalb des Messkanals zu erkennen (siehe US 4,358,423).

Im Experiment wurden neue, nicht verwendete Sensorkassetten erstmalig eingesetzt. Bei diesen Kassetten ist der Sensorkanal trocken und wurde noch nicht mit einer Flüssigkeit in Kontakt gebracht. Es folgt eine Erstbefüllung des Sensorkanals mit einer Testflüssigkeit. Als Testflüssigkeit wurden verwendet: eine Betriebsflüssigkeit (A/B), Blut (C) oder eine Chitosanlösung (D).

Die eingesetzte Betriebsflüssigkeit dient als Kalibrierflüssigkeit und ist eine wässrige Lösung von Salzen und Puffersubstanzen mit eingestellten Konzentrationen an Gasen (O₂/CO₂) und einem pH-Wert von 7,35. Das Monitoring der Gasblasen erfolgt bei der nachfolgenden abwechselnden Befüllung und Entleerung des Sensorkanals mit Betriebsflüssigkeit.

| **Experiment** | **A** | **B** | **C** | **D** |
|---|---|---|---|---|
| **Erstbenetzung** | Erstbenetzung durch Kalibrationslösung | Erstbenetzung durch Kalibrationslösung mit Tensid Triton X 100 | Erstbenetzung durch Blut | Erstbenetzung durch Chitosanlösung pH 6.7 |
| **Betriebsflüssigkeit** | Kalibrationslösung | Kalibrationslösung mit Tensid Triton X 100 | Kalibrationslösung | Kalibrationslösung |
| **Anzahl der beobachteten Zyklen** | 2071 | 1055 | 829 | 832 |
| **Erkannte Luftblasen** | 43% | 6% | 20% | 3% |

Wie aus den Prozentzahlen hervorgeht, zeigt der Tensidzusatz zur Kalibrationslösung eine drastische Reduktion der Luftblasen (Experiment B) (der Tensidzusatz ist aber in der eingesetzten Konzentration nicht realisierbar). Auch die Erstbenetzung mit Blut hat einen positiven Effekt (Experiment C) auf die folgenden Befüllvorgange mit Betriebsflüssigkeit. Dieser Effekt ist bekannt und wahrscheinlich auf die Deposition bestimmter Proteine zurückzuführen. Die Erstbenetzung mit der Chitosanlösung (Experiment D) hat aufgrund der Polymerdeposition eine deutliche Reduktion der Luftblasen zufolge, Die Frequenz der Luftblasenbildung ist ähnlich niedrig, wie bei Experiment B, obwohl die Betriebsflüssigkeit in Experiment D kein Tensid enthält.

## Patentansprüche

1. Verfahren zur zumindest teilweisen Belegung der inneren Oberflächen von fluidischen Komponenten, insbesondere eines zumindest ein Sensorelement aufweisenden Messkanals (5), einer in einen Analysator (1) austauschbar einsetzbaren Sensorkassette (4) mit einem hydrophilen Polymer, **dadurch gekennzeichnet, dass**
a. eine Sensorkassette (4) in den Analysator (1) eingesetzt wird,
b. eine wässrige Lösung eines hydrophilen Polyaminosaccharids in den Messkanal (5) der Sensorkassette (4) eingeleitet wird, sowie
c. die wässrige Lösung des Polyaminosaccharids nach einer Verweilzeit durch ein gasförmiges oder flüssiges Medium ersetzt wird, wobei Reste des Polyaminosaccharids auf der inneren Oberfläche des Messkanals (5) verbleiben und diese hydrophilisieren.

2. Verfahren nach Anspruch 1, **dadurch gekennzeichnet, dass** bereits in Verwendung stehende Sensorkassetten (4) in vorgegebenen Zeitabständen den Schritten b. und c. unterworfen werden.

3. Verfahren nach Anspruch 1 oder 2, **dadurch gekennzeichnet, dass** als Polyaminosaccharid-Lösung Chitosan in wässriger Lösung verwendet wird.

4. Verfahren nach Anspruch 3, **dadurch gekennzeichnet, dass** die wässrige Chitosanlösung einen pH-Wert < 7, vorzugsweise einen pH-Wert zwischen 6,4 und 6,8, aufweist.

5. Verfahren nach Anspruch 3 oder 4, **dadurch gekennzeichnet, dass** die wässrige Chitosanlösung nach einer kurzen Verweilzeit von vorzugsweise 5 bis 30 s durch eine Lösung ersetzt wird die einen im Vergleich zur Chitosanlösung höheren pH-Wert aufweist.

6. Verfahren nach Anspruch 5, **dadurch gekennzeichnet, dass** als Lösung zum Verdrängen der Chitosanlösung eine bevorzugt schwach alkalische Betriebsflüssigkeit des Analysators, beispielsweise eine Wasch-, Kalibrier- oder Qualitätskontrollflüssigkeit verwendet wird.

7. Verfahren nach einem der Ansprüche 3 bis 6, **dadurch gekennzeichnet, dass** die Chitosanlösung aus einem in den Analysator (1) austauschbar einsetzbaren Fluidpack (2) entnommen wird, welches die Betriebsflüssigkeiten, beispielsweise eine Wasch-, Kalibrier- und/oder Qualitätskontrollflüssigkeit, für den Analysator (1) enthält.

8. Sensorkassette (4), die in einen Analysator (1) austauschbar einsetzbar ist, deren innere Oberflächen der fluidischen Komponenten, insbesondere des mindestens ein Sensorelement enthaltendenden Messkanals (5), zumindest teilweise mit einem hydrophilen Polymer belegt ist, **dadurch gekennzeichnet, dass** die inneren Oberflächen einen inselartigen oder vollflächigen Belag aufweisen, der Chitosan enthält oder aus Chitosan besteht.

9. Fluidpack (2), das in einen Analysator (1) einsetzbar ist und mehrere Behältnisse (A, B, C) zur Aufnahme von Wasch-, Kalibrier- und/oder Qualitätskontrollflüssigkeiten für den Analysator (1) aufweist, **dadurch gekennzeichnet, dass** eine der Wasch-, Kalibrier- und/oder Qualitätskontrollflüssigkeiten zusätzlich Chitosan enthält, oder dass das Fluidpack (2) ein weiteres Behältnis (D) zur Aufnahme einer vorzugsweise physiologischen Chitosanlösung enthält.

10. Fluidpack nach Anspruch 9, **dadurch gekennzeichnet, dass** die Chitosankonzentration der Chitosanlösung zwischen 0,03g/l und 3g/l, vorzugsweise bei ca. 0,3 g/l, liegt.

11. Verwendung von Chitosan zur Herstellung eines inselartigen oder vollflächigen Belags auf inneren Oberflächen der fluidischen Komponenten von Sensorkassetten (4), insbesondere eines Messkanals (5) der zumindest ein Sensorelement aufweist.
